# EUROPEAN PATENT SPECIFICATION

(11) **EP 0 327 061 B1**
(45) Date of publication and mention of the grant of the patent: **05.05.1993**
(21) Application number: 89101727.9
(22) Date of filing: 01.02.1989
(51) Int. Cl.: A61M 5/32

(54) **Disposable retractable syringe**
Einziehbare Einwegspritze
Seringue rétractable à usage unique

(30) Priority: 01.02.1988 US 150621; 12.01.1989 US 296495
(43) Date of publication of application: 09.08.1989
(73) Proprietor: THE MEDTECH GROUP, INC., South Plainfield New Jersey 07080 (US)
(72) Inventor: Blake, Joseph Walter III, New Canaan, CT 06840 (US); Sloane, Thomas Edison, Jr., West Reading Connecticut 06896 (US)
(74) Representative: von Samson-Himmelstjerna, Friedrich R., Dipl.-Phys.

(56) References cited:
- EP-A- 0 182 682
- EP-A- 0 282 097
- WO-A-79/01111
- DE-A- 3 346 351
- GB-A- 580 185
- GB-A- 1 228 486
- US-A- 4 675 005
- US-A- 4 747 830

## Description

The present invention relates to a disposable, retractable needle syringe comprising a hollow barrel, disposed about a longitudinal axis, comprising an open proximal end, a substantially closed distal end with a retractable needle extending outwardly, from the barrel, therethrough and means for releasably coupling said needle in its outwardly extended condition, with said barrel distal end; plunger means, axially and reciprocally movable within said barrel, comprising locking means, at its distal end, to engage said needle, decouple it from said, and withdraw it into, said barrel and piston means spaced proximally from said locking means.

The invention furthermore relates to a method for using and safely disposing of a retractable syringe comprising a hollow barrel, disposed about a longitudinal axis, comprising an open proximal end, a substantially closed distal end, with a retractable needle extending outwardly, from the barrel therethrough and means for releasably coupling said needle, in its outwardly extended condition, with said barrel distal end; plunger means, axially and reciprocally movable within said barrel, comprising locking means, at its distal end, to engage said needle, decouple it from said, and withdraw it into, said barrel and piston means spaced proximally from said locking means; said plunger means further comprising spring means according to the preceeding syringe claims.

Health care workers, such as nurses, and even housekeeping personnel are becoming more fearful of exposure to infectious diseases, such as hepatitis and, especially, AIDS, through transmission thereof by accidental impalation of such personnel on hypodermic needles used, e.g., on patients having such diseases.

It has, therefore, become an imperative to provide syringe and needle combinations which will reduce the possibility of such accidents.

Used needle and syringe combinations have also been implicated in drug abuse situations.

It is, therefore, also desirable to provide such combinations which may not easily be reused for such purposes.

Disposable hypodermic needle and syringe combinations, however, must be inexpensive to produce and easy to operate, if they are to be widely utilized to avoid such possibilities.

U.S. Patent 4,592,744 describes such a combination wherein
standard syringe and needle are mounted in a clear plastic sheath. The needle extends through a hole in the bottom of the sheath. The end of the needle is covered with a cap. To use, the cap is removed and the standard medical procedures are carried out in the usual way, but with the syringe still inside of the clear plastic sheath. After use, the syringe and needle are drawn back into the sheath and the needle is completely within the confines of the plastic sheath. Flanges within the sheath catch behind the lip of the needle as the syringe is withdrawn, trapping the needle within the sheath. The needle is thus unable to protrude at either end (Column 2 lines 16 to 28).

The above system suffers from the fact that it requires a separate sheath to contain the used needle. The cost of the combination, which can be reused is, therefore, increased by the requirement for the separate sheath.

Furthermore, if an abuser were to wish to reuse the needle and syringe, it would only be necessary to cut away the sheath and reattach the needle to the syringe.

U.S. Patent 4,702,738 discloses a disposable needle and syringe combination comprising a retractable sheath to cover the needle, after use, and lock in place thereby preventing accidental pricking by the exposed needle or reuse for drug abuse.

This system also suffers from the disadvantages noted above. Thus, if an abuser were to wish to reuse the combination for drug abuse, it would only be necessary to cut through the sheath thereby exposing the needle for reuse.

U.S. Patent 4,747,829 discloses a "Prefilled syringe..." which suffers from the fact that it can only be used in "pre-filled" condition, thus limiting its value. One would be required to have a large number of syringes if one would have many compositions to dispense. Furthermore, one could not use this syringe to withdraw fluids from a source such as a patient.

In addition, the preferred embodiment depends upon a pre-stressed needle which bows out of alignment with the plunger upon withdrawal from the barrel stem. This, of course, creates difficulties in positioning the needle within the syringe.

In U.S. Patent 4,747,830, there is disclosed a retractable syringe wherein the needle is prevented from redescending through the barrel stem, after withdrawal therefrom, by cooperating latching means in the upper portions of the barrel inner wall and the outer wall of the plunger which lock the needle assembly in an elevated position. The latching means are complex and would require expensive tooling.

The Patent also discloses means (see e.g., Figure 15), in the plunger head to engage the needle assembly for removal from the barrel stem. The engagement means 134 would have to break through a wall of a resilient flexible piston 136, which would require considerable force, before it could engage the needle assembly.

Furthermore, at the time, it would be necessary to break through the wall; said wall would be entrapped between the engaging means of the plunger and needle assembly, thereby increasing the difficulty of breaking through it.

It is an object of the invention to provide a hypodermic needle and syringe combination which cannot, readily, be reused for drug abuse after its required use.

This object will be achieved with a disposable, retractable syringe, known from EP-A-0 282 097, representing the features of claim 1's first part, in that said plunger means further comprising spring means, at its distal end, to cause said needle to be canted relative to said longitudinal axis to prevent reextension of the needle through the distal end of the barrel after the needle has been fully drawn into the barrel. Furthermore, this object will be solved in a method for using and safely disposing of a retractable syringe with the features of claim 18's first part by the steps of engaging the complementary engaging means on the lever arm and needle; turning the plunger to disengage the complementary engaging means on the needle and barrel; drawing the plunger proximally until the tip of the needle is in the barrel, whereby the lever arm and needle are canted relative to the longitudinal axis of the barrel and the needle cannot be reextended through the distal opening thereof.

It is another object of the invention to provide a disposable retractable needle syringe which reduces the possibility of infecting persons within its proximity by accidental pricking after use on patients suffering from said diseases.

According to the invention, a needle can be retracted into the barrel of said syringe.

The invention provides a needle and syringe combination, as described above, comprising a syringe assembly comprising a barrel comprising a hollow wall having at its proximal end a large opening to receive a plunger adapted to grip and retract a hypodermic needle and, at its distal end a relatively small opening from which descends a hollow stem adapted to removably receive a needle assembly comprising a hollow tube having a sharp distal end and a hub, adherently surrounding said needle, adapted to be gripped and turned by said plunger and withdrawn from said stem by upward movement of said plunger.

The invention furthermore provides a needle and syringe combination, wherein the axes of the gripping means on the plunger are skewed relative to the longitudinal axis of the plunger but straighten out upon engagement of said means with the complementary means on the needle assembly when said assembly is within the barrel stem but becomes reskewed upon retraction of the assembly from said stem whereby the needle assembly is caused to take an angular position relative to the barrel stem after withdrawal therefrom.

The invention also provides a needle and syringe combination further comprising stopping means to prevent complete withdrawal of the plunger from the barrel after the needle assembly has been withdrawn from the barrel stem. In a further embodiment of the invention said stopping means comprises projections extending into the barrel cavity from the barrel inner wall to engage with cooperating means on the plunger to prevent further outward movement of the plunger. According to a further embodiment of the invention sealing means prevent liquids contained in said barrel from passing between said barrel inner wall and the plunger outer wall.

Another embodiment of the invention provides a syringe which is inexpensive to produce, as it requires no more components than those of the prior art, while providing the extra measure of safety.

According to a further embodiment of the invention, there is provided a syringe which is easy to operate and requires no additional actions, to perform the functions of a syringe, on the part of the user, in that it has no more components, compared to the currently available combinations which do not have its safety features.

Other objects will be in part apparent and part specifically disclosed in connection with the following detailed description and accompanying drawings wherein like numerals indicate like parts.

Figure 1 is a vertical sectional view of the present invention prior to use.

Figure 2 is an exploded vertical sectional view of the present invention after expulsion of the contents thereof.

Figure 3 is a sectional view of the article of Figure 2 along line 2-2 thereof.

Figure 4 is a sectional view of the article of Figure 2 along line 3-3 thereof.

Figure 5 is a vertical sectional view of the upper portion of a second embodiment of the invention.

Figure 6 is a vertical sectional view of the upper portion of a third embodiment of the invention.

Figure 7 is an exploded elevational sectional side view of another embodiment of the invention.

Figure 7b is a 90° rotational sectional view of the portion of Figure 7 indicated by the numeral 3.

Figure 8 is an elevational side view of the portion of the above embodiment indicated by A in Figure 7.

Figure 9 is a 90° rtotational view of the portion of Figure 8.

Figure 10 is a 180° rotational view of the portion of Figure 8.

Figure 11 is a sectional elevational view of the portion of Figure 8 along line 8-8 thereof.

Figure 12 is an elevational sectional side view of the embodiment of Figure 7 before expulsion of fluid therefrom.

Figure 13 is an elevational sectional side view of the embodiment of Figure 7 after expulsion of fluid therefrom.

Figure 14 is an elevational sectional side view of the embodiment of Figure 7 prior to disposal thereof.

### DETAILED DESCRIPTION OF THE PREFERRED EMBODIMENT

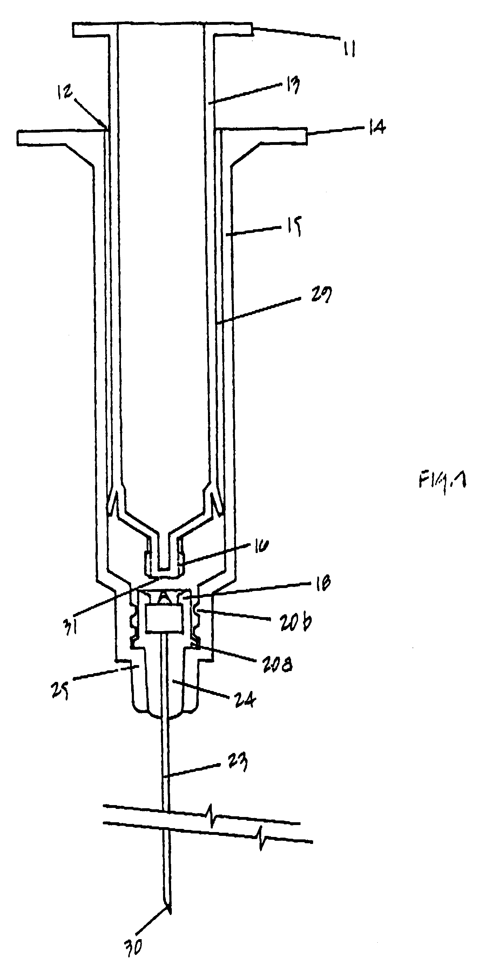

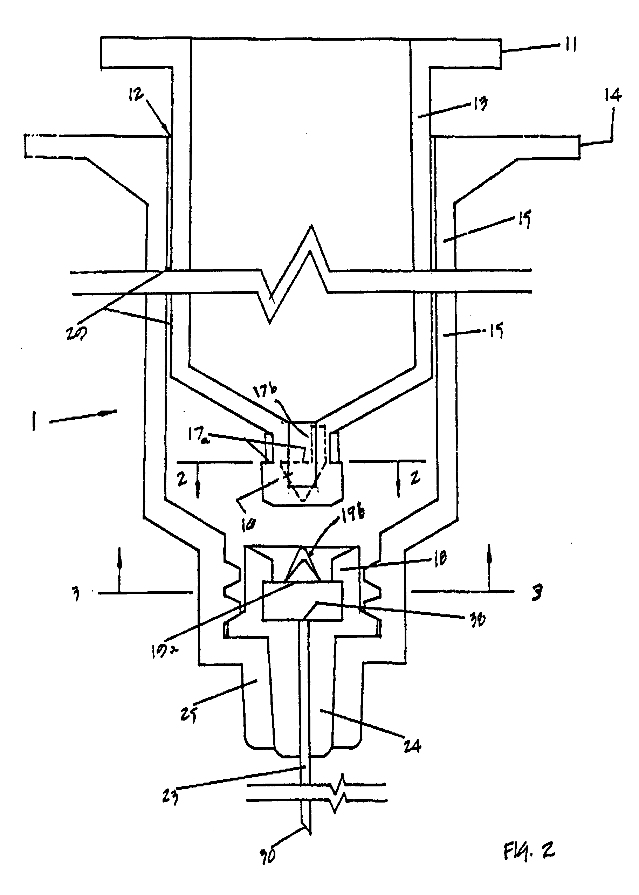

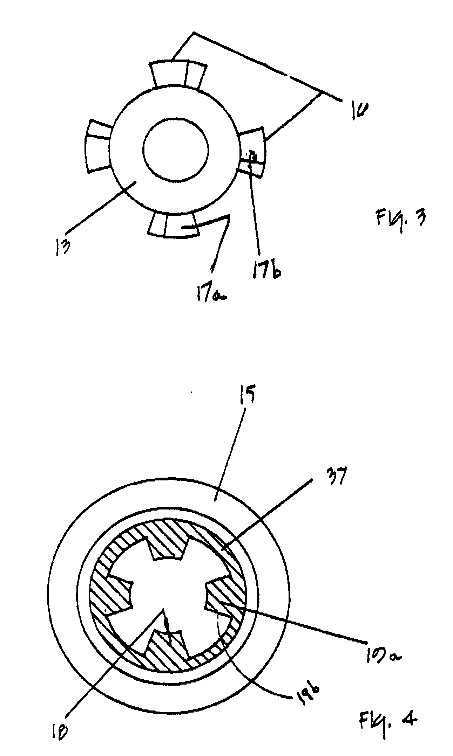

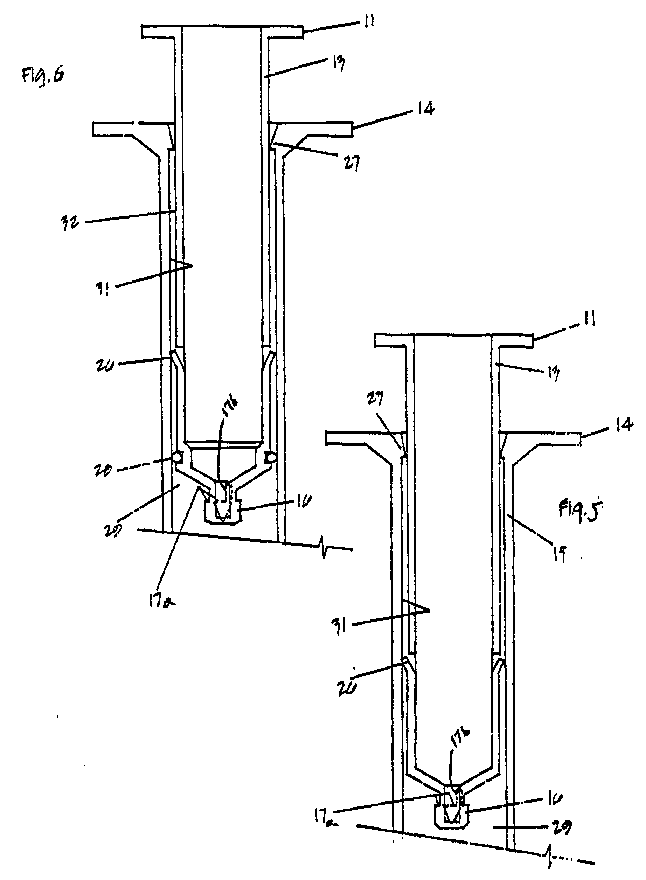

Figures 1-4 illustrate a disposable, retractable hypodermic needle and syringe apparatus generally indicated by the numeral 1. The syringe comprises a barrel comprising an elongated cavity 29 surrounded by a longitudinally extended wall 15 having a large opening 12 at its proximal end and a small opening at its distal end from which there descends vertically a hollow stem 25. Said wall is adapted to removably receive a hypodermic needle assembly.

The syringe further comprises a plunger 13, to be received by said cavity 29, comprising, at its distal end, means to grip and turn said needle assembly when retraction thereof is desired.

Said means comprises radially spaced traingular extensions 16, projecting from the distal end of said plunger, the upper portions of which comprise a notch comprising lower surface 17a and upward directed sides 17b to engage complementary projections 18 of said needle assembly when retraction thereof is required.

The apparatus further comprises a hypodermic needle assembly comprising a hypodermic needle 23 comprising a hollow tube having a sharp distal end 30. Said tube is adherently surrounded by a hub 24 adapted to be received and removably held by the cavity of stem 25.

The hub 24 comprises means, such as threads 20a, spaced from its distal end, which is removably receivable by complementary threads 20b in the wall 15 of the barrel

The proximal end of said hub comprises a circular wall 37 horizontally spaced from the upper opening 38 of needle 23.

Said wall comprises triangular projections 18 having walls 19a and 19b to engage the complementary walls 17a and 17b of the projections 16, of the plunger when retraction of said needle assembly is desired.

In a preferred aspect of the above embodiment said projections 18 also have tapered side walls reaching each other at points at their proximal ends while the projections 16 of the plunger have similar triangular shapes having their pointed junctures at their distal ends.

The above tapered configurations of the walls direct the projections 16 of the plunger past the projections 18 of the needle assembly after which the plunger is turned to engage the walls 17a and 17b and 19a and 19b of the plunger and hub assemblies, respectively, for retraction of the needle assembly.

The above embodiment, however, permits complete withdrawal of the plunger 13 and retracted needle assembly which may then be separated and the apparatus reassembled for possible use by a drug abuser.

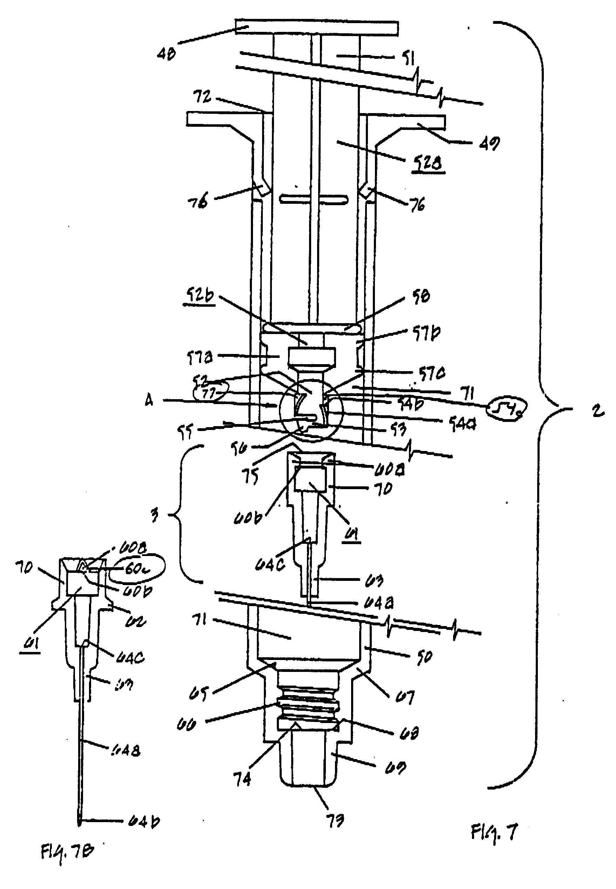

Therefore, in a second embodiment of the invention, as shown in Figure 5 there is provided a disposable, retractable hypodermic needle and syringe apparatus wherein said barrel wall 15 further comprises inwardly-directed projections 27 extending from inner wall 31, near the proximal end, thereof and outwardly-directed projections 26 extending from the outer wall 32, and spaced from the distal end of said plunger 13 the projections being adapted to permit insertion of the plunger 13 into the cavity 29 but on retraction of the needle assembly said projections engage and prevent complete withdrawal of the plunger 13 and needle assembly from the cavity 29 thereby preventing accidental pricking of personnel or reuse by an abuser.

Other means known to the art, including cam extension an indentation systems may also be utilized to prevent such complex withdrawal of the plunger and needle assembly.

As the space between the barrel inner wall 31 and plunger outer wall 32 may be increased, by virtue of the above projections thereon, leakage of the barrel contents therethrough may occur.

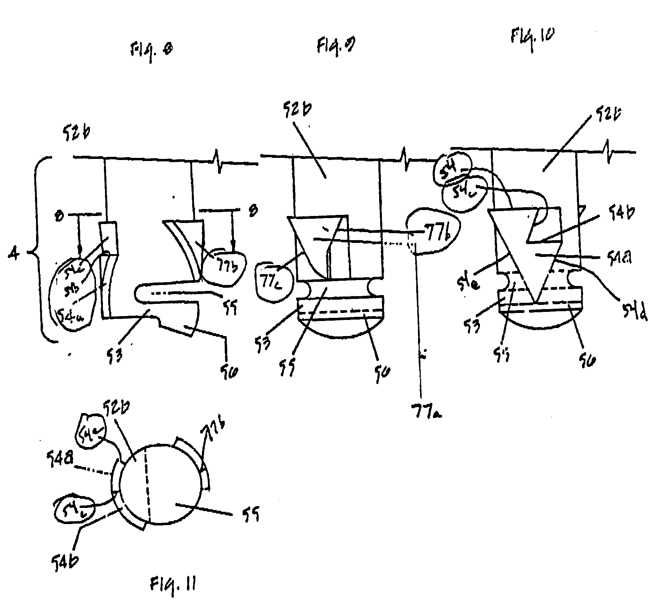

Therefore, in a third embodiment of the invention, illustrated in Figure 6, there are provided sealing means 28 to prevent such leakage. Said means may be permanently or removably affixed to the outer wall 32 of the plunger 13 between its distal end and projections 26. Such means, which are well known in the art, include O-rings inserted into circular grooves in the outer wall of plunger 13. The sealing means 28 may also comprise a separate circular flexible ring sealed to said outer wall or a flange molded into the wall.

The hypodermic needle assembly may be removably held by stem 25 by any means known in the art including complementary threads 20a and 20b, respectively, on the inner wall of stem 25 and the outer wall of hub 24 and Luer connections.

When retraction of the needle is desired the plunger 13 is pushed to the distal end of the barrel whence the upper walls 17 of the projections 16 of the plunger 13 are made to engage the lower surfaces 19 of the projections 18 of the hub 24. The plunger 13 is then turned until the hub 24 is completely disengaged from the stem 25 and drawn back until the hypodermic needle assembly has been withdrawn from the stem 25 and is completely contained within the cavity 29.

In the above embodiments the needle is angled, reelative to the longitudinal axis of the plunger, after retraction of the needle assembly from the stem 69, to assure that reinsertion thereof into stem 25 is prevented. For this purpose the surface 17 and 19 of the projections 16 and 18, respectively, may be skewed so that they are not normal to the axes of the barrel and plunger.

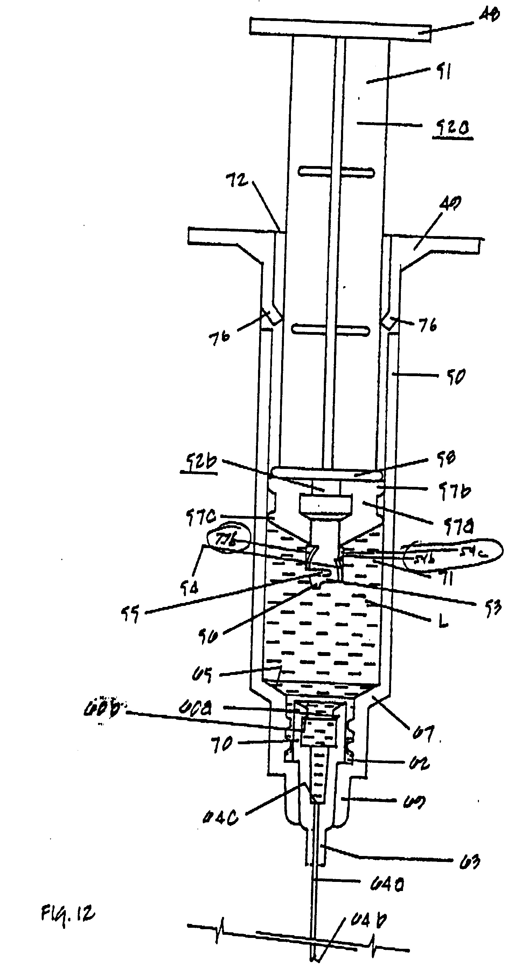

A most preferred embodiment of the invention, as illustrated in Figures 7 and 12-14, is generally indicated by the numeral 2. The syringe comprises a barrel comprising an elongated cavity 71 surrounded by a longitudinally extended wall 50 having a large opening 72 at its proximal end, to receive a needle assembly, and a plunger, and a bottom wall 68 at its distal end comprising a small opening 74 from which there descends vertically a hollow stem 69. The longitudinal wall 50 further comprises an annular protrusion 67 extending horizontally inward from the inner surface of wall 50 and spaced from the lower wall 68 of the barrel. The lower wall 68 and protrusion 67 define an annular groove 66 to removably receive cooperatively acting means on a needle assembly to removably lock the needle assembly into place in the barrel stem 69 prior to use of the syringe. The barrel further comprises a flange 49 extending horizontally outward from the upper end of wall 50 to permit gripping of the syringe by the user.

In the aspect of the invention illustrated herein the wall of the groove 66 comprises a threaded portion to cooperatively receive outwardly-directed projections 62 in the outer wall of the stem of the needle assembly.

The inner surface of the upper portion of barrel wall 50 further comprises stopping projections 76 to prevent complete withdrawal of the plunger from the barrel by engaging cooperative means on the plunger.

The hypodermic needle assembly, indicated by the numeral 3, comprises a cavity 61 surrounded by a circular longitudinal elongated wall 70, a large opening 75 at its upper end to receive the distal end of a plunger and a bottom wall comprising a hub 63 adherently surrounding a hollow tube 64a having a sharp distal end 64b, and a proximal opening 64c spaced horizontally inward from wall 70.

The needle assembly 3 further comprises two projections 62 on opposite sides of, and extending normally outward, from the outer surface of the upper portion of hub 63, to be received in the threaded portion 66 of the barrel when the needle assembly is locked into the stem 69.

Wall 70 comprises, on its inner surface, triangular shaped projections 60a, each having a lower horizontal wall 60b and angular side walls 60c rising from the ends thereof to engage cooperating means on a plunger when locking of the needle assembly 3 into, or withdrawal of the assembly from, the stem 69 of the barrel is desired.

The plunger 51 comprises a longitudinally elongated member 52a having a gripping means 48 at its upper end to permit manipulation, such as turning, of the plunger. At its other end the plunger comprises a plunger head 52b extending from, and of smaller diameter than, member 52a. Member 52a and plunger head 52b are separated from each other by a circular horizontal disc 58 whose outer diameter (OD) is slightly less than the inner diameter (ID) of the barrel cavity 71.

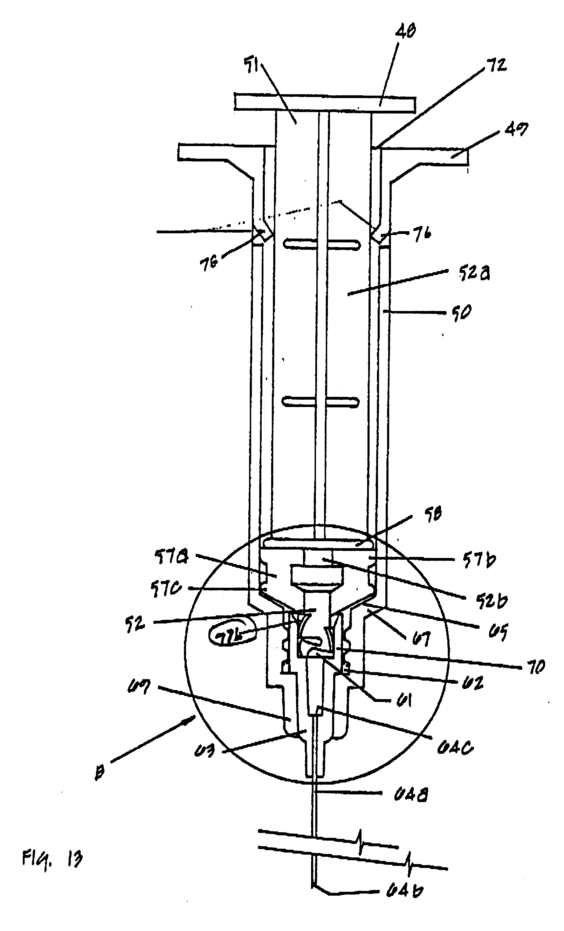

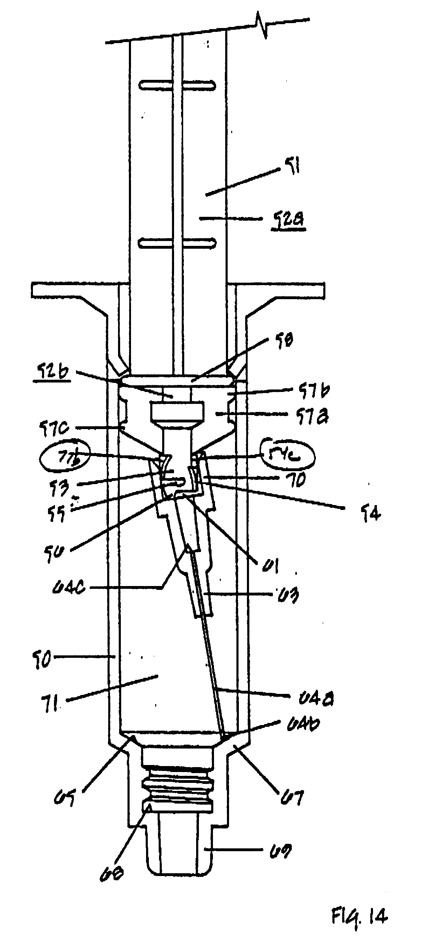

The distal end of the plunger comprises a plunger head 52b, indicated by A in Figure 7 and best discussed in conjunction with Figures 8-11, which comprises a notch 55 extending horizontally partially into the plunger head and a flexible portion 53 terminated by a thin arcuate projection 56, on the same side of the plunger head 52b as notch 55.

Notch 55 and tip portion 53 together form a spring which normally, i.e., when the notch is open, causes the flexible tip to be at an angle relative to the longitudinal axis of the plunger, i.e., when the tip and needle assembly are not engaged or when they are engaged in retraction mode and the needle assembly is not in the barrel stem.

The plunger head 52b further comprises a triangular shaped projection 54a having a horizontal upper wall 54 and first and second angular side walls 54d and 54e descending from the ends thereof said projection being situated on the side of the plunger head opposite the notch 55.

An upper portion of projection 54a is cut away inward from side wall 54d to form a notch comprising an angular side wall 54c, approximately parallel to side wall 54d, and a horizontal wall 54b, said notch being adapted to lockingly receive a part of the lower portion of projection 60a of the needle assembly 3.

It has been found that the projection 54a is sometimes sheared off of plunger head 52b by the force applied thereto when the plunger is being twisted while in locking engagement with the needle assembly when said assembly is being withdrawn from the barrel stem 69.

Accordingly this embodiment further comprises a non-locking triangular projection 77a on the side of plunger head 52b opposite locking projection 54a and above notch 55. This projection distributes the force applied by the plunger head to the needle assembly thereby preventing shearing off of locking projection 54a.

The plunger head 52b, at its proximal end, is surrounded by a semi-flexible tube 57a terminated at its upper 57b and lower 57c ends by horizontal flanges whose ODs are equal to or slightly greater than the ID of the barrel. The upper flange 57b abuts the lower surface of disc 58.

Flanges 57b and 57c provide liquid tight sealing of the cavity 71 from the outside while flange 57c acts as a piston to expel fluid from, or draw fluid into, barrel cavity 71.

In the practice of using this embodiment of the invention the syringe and needle assembly is used as any prior art assembly. It is only with respect to safe disposal that this assembly advantageously differs from the prior art articles.

Thus, the syringe may be obtained prefilled with material to be dispensed or it may be filled by withdrawal of liquids from bodies or containers and the liquids then expelled for disposal or injection.

It is sometimes possible that the plunger, after expulsion of the syringe contents, but prior to retraction of the needle assembly, may not completely fill the space between the walls 37 of the hub. The article of the invention may also, as is commonly done, be used to dispense less than all of the contents of the syringe.

Under those circumstances the barrel may, by known methods, be precalibrated.

After all use of the syringe has been completed and safe disposal thereof required the plunger is fully depressed until its tip 53 enters the cavity 61 of the needle assembly. The notch 55, of the plunger, closes and the tip is caused to align with the longitudinal axis of the plunger thereby causing the spring formed by the notch 55 and tip 53 to be under tension.

The plunger is then turned counter clockwise whereby a part of the lower portion of projection 60a of the needle assembly enters the notch in plunger projection 54a causing a portion of the side 60c and bottom 60b walls of the needle assembly projection to make removably locking contact with side 54c and bottom 54b walls of the plunger projection notch.

Counterclockwise turning of the plunger is continued causing the needle assembly projections 62 to disengage from the threaded groove 66 of barrel stem 69.

The plunger is then withdrawn until further withdrawal thereof is prevented by engagement of barrel wall stopping projections 76 and the upper surface of plunger disc 58.

At that time the tip 64b of needle 64a will have been drawn past projection 67 on the barrel and completely into the barrel cavity 71. The tension in the spring formed by plunger notch 55 and tip 53 is relieved, notch 55 opens and the tip 53 is thrown out of alignment with the plunger longitudinal axis whereby the needle assembly 3 will assume the same angle, relative to said axis, as the tip 53,

Any attempt to cause the needle 64a to be reextended will be frustrated by engagement of the needle tip 64b with the upper portion surface 65 of barrel projection 67.

It is to be understood that the directions of turning of the plunger to achieve the desired results may be reversed upon changing the position of the notch in the plunger projection 54a and side 54c and bottom 54b walls thereof.

Furthermore, other means, as known in the art, may be used to prevent complete withdrawal of the plunger from the barrel.

The articles of the invention may be constructed of any materials known to the art which are compatible with the proposed contents and use of the apparatus.

Preferably the barrel will be constructed of transparent materials, to permit viewing the contents thereof, including glass and plastics such as polyethylene, polypropylene poly(methylpentene), and the like. If desired, the barrel and plunger may be constructed of different materials. For instance, the barrel may be constructed of poly(methylpentene) and the plunger of polypropylene.

The invention has been described, in detail, with respect to specific embodiments. Modifications and variations may be made therein within the scope of the invention as defined by the following claims.

## Claims

1. A disposable retractable needle syringe (2) comprising a hollow barrel (50, 71), disposed about a longitudinal axis, comprising an open proximal end (72), a substantially closed distal end (73), with a retractable needle (64a ... c) extending outwardly, from the barrel (50, 71), therethrough and means for releasably coupling said needle in its outwardly extended condition, with said barrel distal end (73); plunger means (51), axially and reciprocally movable within said barrel (50, 71), comprising locking means, at its distal end, to engage said needle assembly, decouple it from said, and withdraw it into, said barrel and piston means spaced proximally from said locking means and sealing means to prevent leakage from the barrel; said plunger means further comprising spring means, at its distal end, to cause said needle assembly to be canted relative to said longitudinal axis to prevent reextension of the needle (64 a...c) through the distal end (73) of the barrel after the needle (64 a...c) has been fully drawn into the barrel (50, 71); characterized in that said spring means (53, 55) comprises a resilient lever arm whose longitudinal axis is normally canted relative to the longitudinal axis of the barrel (50, 71) when said spring means and needle (64a) are not engaged and said needle is combined with a support to form a needle assembly (60a ... 64c, 70) and that said spring means comprises a cantilever spring which takes up said lever arm in a fixed position.

2. Syringe according to claim 1 characterized in that said plunger means (51) and lever arm comprise a unitary article.

3. Syringe according to any one of claims 1 to 2 characterized in that said needle support means comprises a hub (63) comprising, near the distal end of its outer wall, means (62) to removably engage said hub (63) with complementary engaging means (66) on the barrel (50, 71) prior to and during use, and near the proximal end of its inner wall engaging means (60) to engage said hub (63) with complementary engaging means (52 ..., 56...,77) on the plunger (51), to disengage said assembly (60a ... 64c, 70) from the barrel (50) and retract the needle assembly (60a ... c, 70) into said cavity (71) to prevent reextension of the needle (64 a ... c) through the barrel distal opening (73).

4. Syringe according to any one of the claims 1 to 3 characterized in that said support means and needle comprise a unitary article.

5. Syringe according to one of the preceding claims characterized in that said cantilever spring causes said lever arm to be canted relative to the longitudinal axis of the barrel (50) when said spring is not compressed.

6. Syringe of claim 5 characterized in that said cantilever spring comprises a normally open compressible notch (55) which causes said lever arm to be canted relative to the longitudinal axis of the plunger arm (51) when said notch (55) is open.

7. Syringe of claim 6 characterized in that
a) said lever arm comprises said needle assembly engaging means (52 ... 56, 77) and said needle assembly engaging means (52 ..., 56 ..., 77) comprises a first, triangular, distally-directed projection (54a) on the wall of said lever arm opposite and displaced proximally from said notch (55), to engage complementary engaging means on the inner wall of the cavity of the needle assembly (60a ... 64c, 70) wherein the upper portion of said first projection is partially removed to provide a horizontal surface, or shelf, extending partially through said projection, and a side wall rising from said shelf to the upper horizontal surface of the projection; and
b) said cooperating projections on the inside wall (70) of the needle assembly cavity (61) comprise at least two triangular proximally directed projections (60a) on opposite sides of the cavity wall wherein the lower, flat wall (60b) of one of said projections (60a) can engage the flat wall of the cutaway portion of the first, triangular projection on the plunger head (52b) and one angular wall (60c) of said projection assembly can engage the angular wall of said cutaway portion of the first projection on the plunger tip (53) to facilitate removal of the needle assembly (60a ... 64 c, 70) from the stem (69) and retraction into the barrel cavity (71).

8. Syringe according to claim 7 characterized in that said needle assembly engaging means furhter comprises a second, triangular projection on the wall of the plunger tip (53) opposite to the side on which the first projection is situated and spaced proximally from the notch (55) in said tip (53), whose angular side wall can non-lockingly engage an angular side wall of a second trianular projection (60a) in the cavity (61) of the hub (63) of the needle assembly (60a ... 64c, 70) when one of the projections thereof has engaged the first projection on the plunger tip (53) to facilitate removal of the needle assembly (60a ... 64c, 70) from the stem (69) and retraction into the barrel cavity (71).

9. Syringe according to claim 8 characterized in that said needle assembly engaging means further comprises a thin arcuate (56) projection, extending distally from the lever arm which is canted relative to the longitudinal axis of the barrel (50) when the notch (55) is open and aligned with said axis when the notch (55) is closed.

10. Syringe of any one of the preceding claims characterized in that said sealing and piston means comprise a single unit.

11. Syringe according to any one of the preceding claims 1 to 9 characterized in that said sealing and piston means comprise separate units.

12. Syringe according to any one of claims characterized in that said syringe further comprises complementary stopping means on the plunger (51) and barrel (50) to prevent withdrawal of the needle assembly (60a ... 64c, 70) from the barrel (50).

13. Syringe according to claim 12 characterized in that said stopping means comprises indentation (78) extending inwardly from the proximal portion of the barrel inner wall and annular flange means (58), spaced proximally from said sealing means, on said plunger means (51) whose outer diameter is sufficient to abut said indentation when the upper portion of said plunger means (51) is withdrawn from said cylinder.

14. Syringe according to claims 12 or 13 characterized in that said stopping means on the plunger (51) and said piston means comprise a single unit.

15. A method for using and safely disposing of a disposable retractable needle syringe (2) comprising a hollow barrel (50, 71), disposed about a longitudinal axis, comprising an open proximal end (72), a substantially closed distal end (73), with a retractable needle assembly extending outwardly, from the barrel (50, 71), therethrough and means for releasably coupling said needle assembly comprising a needle (64a ...c) and support means in its outwardly extended condition, with said barrel distal end (73); plunger means (51), axially and reciprocally movable within said barrel (50, 71), comprising locking means, at its distal end, to engage said needle assembly, decouple it from said, and withdraw it into, said barrel and piston means spaced proximally from said locking means and sealing means to prevent leakage from the barrel: said plunger means further comprising spring means, according to the preceding syringe claims the method comprising the steps of:
a) engaging the complementary engaging means on the lever arm and needle assembly;
b) turning the plunger to disengage the complementary engaging means the needle assembly and barrel;
c) drawing the plunger proximally until the tip of the needle is in the barrel whereby the lever arm and needle are canted relative to the longitudinal axis of the barrel and the needle cannot be reextended through the distal opening thereof.

16. The method of claim 15 characterized in that said plunger means and lever arm comprise a unitary article.

17. The method of any one of claims 15 or 16 characterized in that said support means and needle comprise a unitary needle assembly.

18. A method for using and safely disposing of a retractable syringe according to any one of claims 15 to 17 characterized in that said lever arm comprises a cantilever spring which causes said lever arm to be canted relative to the longitunidal axis of the barrel when said spring is not compressed.

19. A method according to claim 18 characterized in that said cantilever spring comprises a compressible notch approximately normal to the longitudinal axis of the lever arm which causes said lever arm to be canted relative to the longitudinal axis of the plunger arm when said notch is open.

## Patentansprüche

1. Einwegspritze (2) mit einziehbarer Nadel, die einen hohlen Zylinder (50, 71) aufweist, der rund um eine Längsachse angeordnet ist und ein im wesentlichen offenes proximales Ende (72), ein im wesentliches geschlossenes distales Ende (73) mit einer sich vom Zylinder (50, 71) hierdurch nach außen erstreckenden einziehbaren Nadel (64a ... c), und eine Einrichtung zum lösbaren Ankuppeln der genannten Nadel in ihrem nach außen ausgefahrenen Zustand an das distale Ende (73) des Zylinders aufweist; eine Stößeleinrichtung (51), die axial und in Gegenrichtungen beweglich im Inneren des genannten Zylinders (50, 71) angeordnet ist und eine Verriegelungseinrichtung an ihrem distalen Ende zum Eingriff mit der genannten Nadelanordnung, zum Entkoppeln vom genannten Zylinder und zum Einziehen in den genannten Zylinder sowie eine Kolbeneinrichtung, die proximal mit Abstand zur genannten Verriegelungseinrichtung angeordnet ist, und eine Dichtungseinrichtung aufweist, um eine Leckage aus dem Zylinder zu vermeiden; die genannte Kolbeneinrichtung weist ferner eine Federeinrichtung an ihrem distalen Ende auf, um die genannte Nadelanordnung zu veranlassen, relativ zur genannten Längsachse schräggestellt zu werden, um das erneute Ausfahren der Nadel (64a ... c) durch das distale Ende (73) des Zylinders zu verhindern, nachdem die Nadel (64a ... c) voll in den Zylinder (50, 71) eingezogen wurde; dadurch **gekennzeichnet,** daß die genannte Federeinrichtung (53, 55) einen federnden Hebelarm aufweist, dessen Längsachse normalerweise relativ zur Längsachse des Zylinders (50, 71) winklig gestellt ist, wenn sich die genannte Federeinrichtung und die Nadel (64a) nicht in Eingriff befinden, daß die genannte Nadel mit einem Träger zur Bildung einer Nadelanordnung (60a ... 64c, 70) kombiniert ist, und daß die genannte Federeinrichtung eine einseitig festgelegte Feder aufweist, die den genannten Hebelarm in eine feste Lage mitnimmt.

2. Spritze nach Anspruch 1, dadurch **gekennzeichnet,** daß die genannte Stößeleinrichtung (51) und der Hebelarm einen einstückigen Gegenstand umfassen.

3. Spritze nach jedem der Ansprüche 1 bis 2, dadurch **gekennzeichnet,** daß die genannte Nadel-Trägereinrichtung eine Buchse (63) aufweist, die nahe dem distalen Ende ihrer Außenwand Mittel (62) zum Herstellen eines lösbaren Eingriffs der genannten Buchse (63) mit einer komplementären Eingriffseinrichtung (66) an dem Zylinder (50, 71) vor und während des Gebrauchs aufweist, sowie nahe dem proximalen Ende ihrer Innenwand eine Eingriffseinrichtung (60), um den Eingriff der genannten Buchse (63) mit einer komplementären Eingriffseinrichtung (52 ..., 56 ..., 77) am Stößel (51) herzustellen, um die genannte Anordnung (60a ... 64c, 70) aus dem Eingriff mit dem Zylinder (50) zu lösen und die Nadelanordnung (60a ... c, 70) in den genannten Hohlraum (71) einzuziehen, um das erneute Ausfahren der Nadel (64a ... c) durch die distale Zylinderöffnung (73) zu verhindern.

4. Spritze nach irgendeinem der Ansprüche 1 bis 3, dadurch **gekennzeichnet,** daß die genannte Trägereinrichtung und Nadel einen einheitlichen Gegenstand umfassen.

5. Spritze nach einem der vorangehenden Ansprüche, dadurch **gekennzeichnet,** daß die gennante, einseitig festgelegte Feder den Hebelarm veranlaßt, relativ zur Längsachse des Zylinders (50) gekippt zu werden, wenn die Feder nicht zusammengedrückt ist.

6. Spritze nach Anspruch 5, dadurch **gekennzeichnet,** daß die genannte, einseitig festgelegte Feder eine normalerweise offene, zusammendrückbare Kerbe (55) aufweist, welche den genannten Hebelarm veranlaßt, relativ zur Längsachse des Stößelarmes (51) gekippt zu werden, wenn die genannte Kerbe (55) offen ist.

7. Spritze nach Anspruch 6, dadurch **gekennzeichnet,** daß
a) der genannte Hebelarm die genannte Nadelanordnungs-Eingriffseinrichtung (52 ... 56, 77) aufweist und die genannte Nadelanordnungs-Eingriffseinrichtung (52 ..., 56 ..., 77) einen ersten, dreieckigen, distal ausgerichteten Vorsprung (54a) an der Wand des genannten Hebelarms der genannten Kerbe (55) gegenüberliegend und proximal gegenüber dieser versetzt aufweist, um in eine komplementäre Eingriffseinrichtung an der Innenwand des Hohlraums der Nadelanordnung (60a ... 64c, 70) einzugreifen, worin der obere Abschnitt des genannten ersten Vorsprungs teilweise entfernt ist, um eine horizontale Fläche oder einen Sims zu liefern, die bzw. der sich teilweise durch den genannten Vorsprung erstreckt, und eine Seitenwand, die vom genannten Sims zur oberen horizontalen Fläche des Vorsprungs ansteigt; und
b) die zusammenwirkenden Vorsprünge an der innenseitigen Wand (70) des Nadelanordnungs-Hohlraums (61) mindestens zwei dreieckige, proximal ausgerichtete Vorsprünge (60a) an den gegenüberliegenden Seiten der Hohlraumwand aufweisen, worin die untere, flache Wand (60b) eines der genannten Vorsprünge (60a) in die flache Wand des weggeschnittenen Abschnitts des ersten, dreieckigen Vorsprungs am Stößelkopf (52b) eingreifen kann und eine winklige Wand (60c) der genannten Vorsprungsanordnung in die winklige Wand des genannten weggeschnittenen Abschnitts des ersten Vorsprungs an der Stößelspitze (53) eingreifen kann, um das Entfernen der Nadelanordnung (60a ... 64c, 70) vom Schaft (69) und das Einziehen in den Zylinderhohlraum (71) zu erleichtern.

8. Spritze nach Anspruch 7, dadurch **gekennzeichnet,** daß die genannte Nadelanordnungs-Eingriffseinrichtung ferner einen zweiten, dreieckigen Vorsprung an der Wand der Stößelspitze (53) gegenüber der Seite, an welcher der erste Vorsprung gelegen ist, und mit proximalem Abstand zur Kerbe (55) in der genannten Spitze (53) aufweist, dessen winklige Seitenwand in nicht verriegelnden Eingriff mit einer winkligen Seitenwand eines zweiten dreieckigen Vorsprungs (60a) im Hohlraum (61) der Buchse (63) der Nadelanordnung (60a ... 64c, 70) treten kann, wenn einer der Vorsprünge hiervon in den ersten Vorsprung an der Stößelspitze (53) eingegriffen hat, um die Entfernung der Nadelanordnung (60a ... 64c, 70) vom Schaft (69) und das Einziehen in den Zylinderhohlraum (71) zu erleichtern.

9. Spritze nach Anspruch 8, dadurch **gekennzeichnet,** daß die genannte Nadelanordnungs-Eingriffseinrichtung ferner einen dünnen bogenförmigen (56) Vorsprung aufweist, der sich vom Hebelarm aus distal erstreckt, welcher relativ zur Längsachse des Zylinders (50) schräggestellt ist, wenn die Kerbe (55) offen ist, und ausgerichtet ist auf die genannte Achse, wenn die Kerbe (55) geschlossen ist.

10. Spritze nach irgendeinem der vorangehenden Ansprüche, dadurch **gekennzeichnet,** daß die genannte Dichtungs- und Kolbeneinrichtung eine einzige Einheit umfaßt.

11. Spritze nach irgendeinem der vorangehenden Ansprüche 1 bis 9, dadurch **gekennzeichnet,** daß die genannte Dichtungs- und Kolbeneinrichtung getrennte Einheiten umfaßt.

12. Spritze nach irgendeinem der Ansprüche, dadurch **gekennzeichnet,** daß die genannte Spritze ferner komplementäre Anschlagmittel am Stößel (51) und am Zylinder (50) aufweist, um das Herausziehen der Nadelanordnung (60a ... 64c, 70) aus dem Zylinder (50) zu verhindern.

13. Spritze nach Anspruch 12, dadurch **gekennzeichnet,** daß die genannte Anschlageinrichtung eine Vertiefung (78) aufweist, die sich vom proximalen Abschnitt der Zylinder-Innenwand aus nach innen erstreckt, und eine ringförmige Flanscheinrichtung (58), die proximal mit Abstand zur Dichtungseinrichtung an der genannten Stößeleinrichtung (51) angeordnet ist, deren Außendurchmesser ausreicht, um gegen die Vertiefung anzuschlagen, wenn der obere Abschnitt der genannten Stößeleinrichtung (51) aus dem genannten Zylinderkörper herausgezogen wird.

14. Spritze nach Anspruch 12 oder 13, dadurch **gekennzeichnet,** daß die genannte Anschlageinrichtung am Stößel (51) und an der genannten Kolbeneinrichtung eine einzige Einheit umfaßt.

15. Verfahren zur Benutzung und zum sicheren Wegwerfen einer Einwegspritze (2) mit einziehbarer Nadel, die einen hohlen Zylinder (50, 71) aufweist, der rund um eine Längsachse angeordnet ist und ein im wesentlichen offenes proximales Ende (72), ein im wesentliches geschlossenes distales Ende (73) mit einer sich vom Zylinder (50, 71) hierdurch nach außen erstreckenden einziehbaren Nadel (64a ... c), und eine Einrichtung zum lösbaren Ankuppeln der genannten Nadel in ihrem nach außen ausgefahrenen Zustand an das distale Ende (73) des Zylinders aufweist; eine Stößeleinrichtung (51), die axial und in Gegenrichtungen beweglich im Inneren des genannten Zylinders (50, 71) angeordnet ist und eine Verriegelungseinrichtung an ihrem distalen Ende zum Eingriff mit der genannten Nadelanordnung, zum Entkoppeln vom genannten Zylinder und zum Einziehen in den genannten Zylinder sowie eine Kolbeneinrichtung, die proximal mit Abstand zur genannten Verriegelungseinrichtung angeordnet ist, und eine Dichtungseinrichtung aufweist, um eine Leckage aus dem Zylinder zu vermeiden; wobei die genannte Stößeleinrichtung ferner eine Federeinrichtung aufweist gemäß den vorangehenden Spritzenansprüchen, wobei das Verfahren die folgenden Schritte aufweist:
a) Herstellen des Eingriffs der komplementären Eingriffseinrichtungen an dem Hebelarm und der Nadelanordnung;
b) Drehen des Stößels zum Lösen des Eingriffs der komplementären Eingriffseinrichtung der Nadelanordnung und des Zylinders;
c) Ziehen am Stößel in proximaler Richtung, bis die Spitze der Nadel sich im Zylinder befindet, wobei der Hebelarm und die Nadel relativ zur Längsachse des Zylinders gekippt werden und die Nadel durch dessen distale Öffnung nicht mehr wieder ausgefahren werden kann.

16. Verfahren nach Anspruch 15, dadurch **gekennzeichnet,** daß die genannte Stößeleinrichtung und der Hebelarm einen einheitlichen Artikel umfassen.

17. Verfahren nach irgendeinem der Ansprüche 15 und 16, dadurch **gekennzeichnet,** daß die genannte Trägereinrichtung und Nadel eine einheitliche Nadelanordnung umfassen.

18. Verfahren zur Benutzung und zum sicheren Wegwerfen einer einziehbaren Spritze nach irgendeinem der Ansprüche 15 bis 17, dadurch **gekennzeichnet,** daß der genannte Hebelarm eine einseitig festgelegte Feder aufweist, die den Hebelarm veranlaßt, relativ zur Längsachse des Zylinders gekippt zu werden, wenn die genannte Feder nicht zusammengedrückt ist.

19. Verfahren nach Anspruch 18, dadurch **gekennzeichnet,** daß die genannte, einseitig festgelegte Feder eine zusammendrückbare Kerbe etwa senkrecht zur Längsachse des Hebelarms aufweist, die den genannten Hebelarm veranlaßt, relativ zur Längsachse des Stößelarmes gekippt zu werden, wenn die genannte Kerbe offen ist.

## Revendications

1. Seringue (2) à usage unique pour aiguille rétractable, comprenant un corps creux (50, 71) d'axe longitudinal et comportant une extrémité proximale ouverte (72), une extrémité distale sensiblement fermée (73), une aiguille rétractable (64a...c) s'étendant vers l'extérieur à partir du corps (50, 71), et des moyens pour coupler de façon détachable ladite aiguille dans la position où elle s'étend vers l'extérieur à partir de l'extrémité distale du corps ; un piston (51) mobile suivant les directions axiales dans ledit corps (50, 71) et comportant des moyens de verrouillage à son extrémité distale pour engager l'ensemble à aiguille, le désaccoupler et le retirer, lesdits corps et piston étant disposés à proximité desdits moyens de verrouillage, des organes d'étanchéité pour empêcher une fuite à partir du corps ; le piston comprend en outre des moyens élastiques, à son extrémité distale, pour permettre, à l'ensemble à aiguille d'être incliné par rapport à l'axe longitudinal de manière à empêcher une réextension de l'aiguille (64a...c) à travers l'extrémité distale (73) du corps après que l'aiguille (64a...c) ait été complètement tirée dans le corps (50, 71) ; caractérisée en ce que les moyens élastiques (53, 55) comportent un bras de levier souple dont l'axe longitudinal est normalement incliné par rapport à l'axe longitudinal du corps (50, 71) lorsque lesdits moyens élastiques et l'aiguille (64a) ne sont pas engagés, ladite aiguille étant combinée avec un support pour constituer un ensemble à aiguille (60a,... 64c, 70), et en ce que les moyens élastiques comprennent un ressort cantilever qui amène ledit bras de levier dans une position fixe.

2. Seringue selon la revendication 1, caractérisée en ce que le piston (51) et le bras de levier sont d'une seule pièce.

3. Seringue selon l'une des revendications 1 ou 2, caractérisée en ce que le support d'aiguille comprend un moyeu (63) qui est muni, près de l'extrémité distale de sa paroi externe, de moyens (62) pour engager de manière amovible ledit moyeu aux moyens d'engagement complémentaires (66) du corps (50, 71) avant et pendant l'utilisation de ladite seringue, et près de l'extrémité proximale de sa paroi interne, de moyens d'engagement (60) pour engager ledit moyeu (63) avec des moyens d'engagement complémentaires (52..., 56..., 57) du piston (51), désaccoupler ledit ensemble (60a...64c, 70) du corps (50) et rétracter l'ensemble à aiguille (60a...c, 70) dans la cavité (71) de manière à empêcher une réextension de l'aiguille (64a...c) à travers l'ouverture distale (73) du corps.

4. Seringue selon l'une des revendications 1 à 3, caractérisée en ce que le support et l'aiguille sont d'une seule pièce.

5. Seringue selon l'une des revendications précédentes, caractérisée en ce que le ressort cantilever amène le bras de levier à être incliné par rapport à l'axe longitudinal du corps (50) lorsque ledit ressort n'est pas comprimé.

6. Seringue selon la revendication 5, caractérisée en ce que le ressort cantilever comporte une encoche (55) normalement ouverte qui amène ledit bras de levier à être incliné par rapport à l'axe longitudinal de la tige de piston (51) lorsque l'encoche est ouverte.

7. Seringue selon la revendication 6, caractérisée en ce que :
a. le bras de levier comprend lesdits moyens d'engagement (52..., 56, 77) de l'ensemble à aiguille, lesdits moyens d'engagement (52..., 56..., 77) comportant, sur le côté du bras de levier opposé et espacé de l'encoche (55), une première projection (54a) qui est triangulaire et située vers l'extrémité distale, de manière à coopérer avec les moyens d'engagement complémentaires de la paroi interne de la cavité de l'ensemble à aiguille (60a...64c, 70), la partie supérieure de ladite projection étant partiellement dégagée pour réaliser une surface horizontale ou appui qui s'étend partiellement sur ladite projection et un côté latéral partant dudit appui vers la surface horizontale supérieure de la projection ; et
b. les projections coopérantes de la paroi interne (70) de la cavité (61) de l'ensemble à aiguille comprennent au moins deux projections triangulaires (60a) dirigées vers la partie proximale et situées sur les côtés opposés de la paroi de la cavité, le côté plat et inférieur (60b) de l'une desdites projections (60a) pouvant coopérer avec le côté plat de la partie coupée de la première projection triangulaire de la tête de piston (52b), un côté incliné (60c) de l'ensemble de projections pouvant coopérer avec le côté incliné de la partie coupée de la première projection de l'extrémité du piston (53) de manière à faciliter la séparation de l'ensemble à aiguille (60a...64c, 70) de la tige (69) et la rétraction dans la cavité (71) du corps.

8. Seringue selon la revendication 7, caractérisée en ce que les moyens d'engagement de l'ensemble à aiguille comprennent, en outre, une deuxième projection triangulaire sur le côté de l'extrémité du piston (53) qui est opposé au côté sur lequel est située la première projection, ladite deuxième projection étant située proche de l'encoche (55) dont le côté latéral incliné ne peut se verrouiller sur le côté latéral d'une deuxième projection (60a) dans la cavité (61) du moyeu (63) de l'ensemble à aiguille (60a...64c, 70) lorsqu'une de ses projections coopère avec la première projection de l'extrémité du piston (53) de manière à faciliter la séparation de l'ensemble à aiguille (60a...64c, 70) d'avec la tige (69) et la rétraction dans la cavité (71) du corps.

9. Seringue selon la revendication 8, caractérisée en ce que les moyens d'engagement de l'ensemble à aiguille comprennent, en outre, une projection courbe et mince (56) qui s'étend de l'extrémité distale du bras de levier qui est incliné par rapport à l'axe longitudinal du corps (50) lorsque l'encoche (55) est ouverte et alignée avec ledit axe lorsque l'encoche (55) est fermée.

10. Seringue selon l'une des précédentes revendications, caractérisée en ce que le piston et les organes d'étanchéité sont d'une seule pièce.

11. Seringue selon l'une des revendications précédentes, caractérisée en ce que les organes d'étanchéité et le piston sont en plusieurs pièces distinctes.

12. Seringue selon l'une des revendications précédentes, caractérisée en ce que ladite seringue comprend, en outre, des moyens d'arrêt complémentaires prévus sur le piston (51) et le corps (50) pour empêcher un retrait de l'ensemble à aiguille (60a...64c, 70) du corps (50).

13. Seringue selon la revendication 12, caractérisée en ce que les moyens d'arrêt comprennent une indentation (76) s'étendant vers l'intérieur à partir de la partie proximale de la paroi interne du corps et des rebords annulaires (57) qui sont espacés des organes d'étanchéité et qui sont disposés sur le piston (51) dont le diamètre est suffisant pour venir en appui sur ladite indentation lorsque la partie supérieure du piston (51) est retirée dudit cylindre.

14. Seringue selon les revendications 12 ou 13, caractérisée en ce que les moyens d'arrêt du piston (51) sont réalisés d'une seule pièce avec ledit piston.

15. Procédé pour utiliser et disposer de façon sûre d'une seringue (2) à usage unique, à aiguille rétractable et qui comprend un corps creux (50, 71) qui est disposé sur un axe longitudinal et qui comporte une extrémité proximale ouverte (72) et une extrémité distale fermée (73), un ensemble à aiguille rétractable s'étendant dans et à l'extérieur du corps (50, 71), des moyens pour coupler de façon détachable l'ensemble à aiguille qui comprend une aiguille (64a...c) et un support dans sa position s'étendant à l'extérieur par rapport à ladite extrémité distale (73) du corps ; un piston (51) qui est mobile dans les deux directions axiales dans ledit corps (50, 71), le piston comportant des moyens de verrouillage à son extrémité distale pour coopérer avec ledit ensemble à aiguille, le découpler de et le tirer dans ledit corps, ledit piston étant disposé près des moyens de verrouillage, et des organes d'étanchéité pour empêcher une fuite à partir du corps, le piston comportant, en outre, des moyens élastiques selon les revendications précédentes concernant la seringue, le procédé comprenant les étapes de :
a. coopération entre les moyens d'engagement du bras de levier avec l'ensemble à aiguille ;
b. rotation du piston pour désaccoupler les moyens d'engagement complémentaires de l'ensemble à aiguille du corps ;
c. déplacement du piston jusqu'à ce que l'extrémité de l'aiguille soit à l'intérieur du corps, de façon que le bras de levier et l'aiguille soient inclinés par rapport à l'axe longitudinal du corps et que l'aiguille ne puisse être réintroduite dans l'extrémité distale dudit corps.

16. Procédé selon la revendication 15, caractérisé en ce que le piston et le bras de levier sont d'une seule pièce.

17. Procédé selon les revendications 15 ou 16, caractérisé en ce que le support et l'aiguille constituent un ensemble à aiguille unitaire.

18. Procédé pour utiliser et disposer de façon sûre d'une seringue rétractable selon l'une quelconque des revendications 15 à 17, caractérisé en ce que le bras de levier comprend un ressort cantilever qui amène le bras de levier à être incliné par rapport à l'axe longitudinal du corps lorsque le ressort n'est pas comprimé.

19. Procédé selon la revendication 18, caractérisé en ce que le ressort cantilever comprend une encoche comprimable sensiblement à la normale de l'axe longitudinal du bras de levier, ce qui amène ledit bras de levier à être incliné par rapport à l'axe longitudinal de la tige de piston lorsque ladite encoche est ouverte.
